# EUROPEAN PATENT APPLICATION

(11) **EP 2 993 166 A1**
(43) Date of publication of application: **09.03.2016**
(21) Application number: 14791804.9
(22) Date of filing: 22.04.2014
(51) Int. Cl.: C07C 315/06, C07C 317/04

(54) **METHOD FOR PURIFYING DIMETHYL SULFOXIDE**

(30) Priority: 30.04.2013 JP 2013095375
(71) Applicant: Toray Fine Chemicals Co., Ltd., Tokyo 101-0041 (JP)
(72) Inventor: ISHINO, Michiko, Tokai-shi Aichi 476-8567 (JP); TANAKA, Koji, Moriyama-shi Shiga 524-0041 (JP); ABE, Hiromitsu, Tokai-shi Aichi 476-8567 (JP); INOUE, Hideki, Moriyama-shi Shiga 524-0041 (JP)
(74) Representative: Kador & Partner
(86) International application number: PCT/JP2014/061306
(87) International publication number: WO 2014/178309

(57) **Abstract**

The present invention provides a method for purifying dimethyl sulfoxide economically and at good yield so as to satisfy the specifications of the United States Pharmacopoeia and reduce odor. The method is characterized in that a solution having 35 parts by weight or more of water admixed per 100 parts by weight of raw dimethyl sulfoxide is distilled.

## Description

### Technical Field

The present invention relates to a method for purifying dimethyl sulfoxide, which is widely used as reaction solvent and synthesis reagent for medical and agrochemical intermediates and the like, etc., and more specifically relates to a method for economical, high-yield purification to provide high-quality dimethyl sulfoxide that is conformable to the U.S. Pharmacopeia and free of significant odors.

### Background Art

Dimethyl sulfoxide has been widely used in industrial fields as reaction solvent and synthesis reagent for medical and agrochemical intermediates, special detergents for electronic materials, and the like. In particular, those dimethyl sulfoxide products specified in the U.S. Pharmacopeia are required to be higher in purity than the dimethyl sulfoxide products with quality for industrial use and also required to be free of significant odors.

Conventionally known methods for purifying dimethyl sulfoxide include a purification method in which dimethyl sulfoxide of general industry grade quality is brought into contact with activated carbon, followed by distillation treatment (see, for example, Patent document 1) and a purification method in which dimethyl sulfoxide of general industry grade quality is mixed with an organic solvent such as toluene to prepare a solution, followed by adding water, extracting dimethyl sulfoxide from the organic phase, and distillation (see, for example, Patent document 2).

However, the purification method proposed in Patent document 1 cannot remove odors. Although not specified in the U.S. Pharmacopeia, low odor emission is substantially an essential quality requirement for dimethyl sulfoxide products for medical use.

As dimethyl sulfoxide is highly soluble in both water and organic solvents, the purification method proposed in Patent document 2 is likely to suffer a large loss in the extraction step. Accordingly, a large quantity of water is necessary to reduce the loss and this large quantity of water has to be removed by distillation treatment, making the process uneconomical. Furthermore, although dimethyl sulfoxide products as specified in the Pharmacopoeia are administered directly to the human body, the purification method proposed in Patent Document 2 uses organic solvents such as toluene that are harmful to the human body, leaving safety concerns about their use in drugs listed in the Pharmacopoeia.

### Prior Art Documents

### Patent Documents

Patent document 1: Japanese Examined Patent Publication (Kokoku) No. SHO 43-12128
Patent document 2: U.S. Patent No. 3,358,036

### Summary of the Invention

### Problems to be Solved by the Invention

An object of the present invention is to solve these problems and provide a method for economical, high-yield purification to provide high-quality dimethyl sulfoxide that is conformable to the U.S. Pharmacopeia and free of significant odors.

### Means of Solving the Problems

The dimethyl sulfoxide purification method that solve the problems according to the present invention and high-purity dimethyl sulfoxide have the following constitution.
(1) A method for purifying dimethyl sulfoxide including a step for distilling a mixed solution containing 35 parts by weight or more of water and 100 parts by weight of dimethyl sulfoxide.
(2) A method for purifying dimethyl sulfoxide as described in paragraph (1) wherein the purified dimethyl sulfoxide has an absorbance of 0.01 to 0.20 at 275 nm.
(3) A method for purifying dimethyl sulfoxide as described in either paragraph (1) or (2) wherein the water is ion-exchanged water, distilled water, or ultrapure water.
(4) A method for purifying dimethyl sulfoxide as described in any one of paragraphs (1) to (3) wherein the water has an electrical conductivity of 50 mS/m or less at 25°C.
(5) A method for purifying dimethyl sulfoxide as described in any one of paragraphs (1) to (4) wherein the distillation of a mixed solution is carried out in a single step for evaporating water to obtain dimethyl sulfoxide.
(6) A method for purifying dimethyl sulfoxide as described in any one of paragraphs (1) to (4) wherein the distillation of a mixed solution is carried out in a first step for evaporating water to obtain roughly purified dimethyl sulfoxide, followed by a second step for distilling the roughly purified dimethyl sulfoxide to obtain purified dimethyl sulfoxide.
(7) High-purity dimethyl sulfoxide purified by a purification method as described in any one of paragraphs (1) to (6) and having a purity of 99.996% or more as determined by gas chromatograph (GC) analysis.

### Advantageous Effect of the Invention

The dimethyl sulfoxide purification method according to the present invention serves to improve the absorbance, which represents a requirement specified in the U.S. Pharmacopeia, and largely decrease the odor emission, which represents a de facto quality requirement. Only water, which can be easily separated by distillation, is mixed with the dimethyl sulfoxide, making it possible to obtain a high-purity dimethyl sulfoxide with a high yield. In addition, the process is very economical because it does not require a large quantity of water.

### Description of Preferred Embodiments

The dimethyl sulfoxide purification method according to the present invention meets the relevant requirements of the U.S. Pharmacopeia and serves for economical, high-yield production of high-quality dimethyl sulfoxide free of significant odor emission. As stated above, when a conventional method is used to purify dimethyl sulfoxide of general industry grade, the purified dimethyl sulfoxide will still maintain unremoved odors and will be difficult to apply to medical uses even if it meets the relevant requirements of the U.S. Pharmacopeia. A process with low productivity and high purification cost as described in Patent document 2 has been necessary to remove odors.

In this connection, the U.S. Pharmacopeia (U.S. Pharmacopeia USP36, NF31, p.3269) specifies the absorbance at 275 nm as a quality requirement and European Pharmacopeia (European Pharmacopoeia 7.0) also specifies the absorbance at 275 nm. The difference in the absorbance at 275 nm represents the largest difference between general industry grade products and Pharmacopoeia-conformable ones and accordingly, the absorbance at 275 nm as specified in the Pharmacopoeia is used herein as a primary quality item in quality evaluation of dimethyl sulfoxide. Determination of the absorbance uses an ultraviolet absorptiometer, a cell with a width of a 1 cm, and water as blank. A dimethyl sulfoxide specimen is first subjected to nitrogen bubbling for 30 minutes and measurement is performed within 10 minutes.

The odor of dimethyl sulfoxide is ranked according to the six-stage odor intensity criterion that has been used for odor evaluation in the Offensive Odor Control Act etc. This odor intensity ranking method ranks the odor intensity as 0 to 5 according to the six-stage odor intensity criterion shown below. The odor level of dimethyl sulfoxide can be evaluated according to this method.
Odor intensity 0: odorless
Odor intensity 1: barely perceivable odor
Odor intensity 2: identifiable but weak order
Odor intensity 3: easily perceivable odor
Odor intensity 4: strong odor
Odor intensity 5: intense odor

To determine the odor of dimethyl sulfoxide, 15 mL of a dimethyl sulfoxide specimen was taken in a 30 mL sample bottle and the nose is brought close to the sample bottle at room temperature. Three or more testers sniff the odor from the same specimen and the rank agreed on by the largest number of testers was taken as the result of the odor intensity test.

There are no specific limitations on the raw materials, production method, and producer country of the dimethyl sulfoxide to be treated by the dimethyl sulfoxide purification method according to the present invention (hereinafter referred to as raw dimethyl sulfoxide), but preferably, it is dimethyl sulfoxide of general industry grade. There are no specific limitations on the purity of raw dimethyl sulfoxide, but it is preferably 99 wt% or more, more preferably 99.9 to 100 wt%. The use of raw dimethyl sulfoxide with such a purity serves to provide high-quality dimethyl sulfoxide that is low in impurities content, conformable to the U.S. Pharmacopeia, and free of odors. For the present invention, the purity of dimethyl sulfoxide is measured with a gas chromatograph according to the method specified in the U.S. Pharmacopeia (U.S. Pharmacopeia USP36, NF31, p. 3269). Raw dimethyl sulfoxide may contain a compound other than dimethyl sulfoxide as long as it can be separated from the dimethyl sulfoxide by distillation.

The dimethyl sulfoxide purification method according to the present invention includes a step for distilling a mixed solution containing 35 parts by weight or more of water and 100 parts by weight of raw dimethyl sulfoxide. Mixing the raw dimethyl sulfoxide with water facilitates the removal of impurities existing in trace amounts in the raw dimethyl sulfoxide and particularly serves to reduce the odor intensity significantly. Reduction in the absorbance and odor of dimethyl sulfoxide cannot be easily realized if the content of water is not appropriate or if a solvent other than water is contained.

The water to be mixed with the raw dimethyl sulfoxide may be of any type that is generally available and it may be, for example, tap water, industrial water, pure water, steam-condensed water, distilled water, ion-exchanged water, or ultrapure water. The water is preferably conformable to A4 of JIS-K0557. Examples of water conformable to A4 of JIS-K0557 include distilled water, ion-exchanged water, and ultrapure water.

The water to be used preferably has an electrical conductivity of 50 mS/m or less, more preferably 30 mS/m or less, at 25°C. The use of water having such an electrical conductivity serves to reduce the odor intensity of dimethyl sulfoxide. Generally, water conformable to A4 of JIS-K0557 has an electrical conductivity of 0.1 mS/m (25°C). For the present invention, the electrical conductivity is measured at 25°C according to JIS-K0130.

In regard to the addition of water to the raw dimethyl sulfoxide, the water should account for 35 parts by weight or more, preferably 50 parts by weight or more, relative to 100 parts by weight of the raw dimethyl sulfoxide. The odor reducing effect cannot be developed sufficiently if the content of water is smaller than 35 parts by weight. The upper limit of the water content is preferably 500 parts by weight, more preferably 300 parts by weight. If the quantity of water addition is more than 500 parts by weight, the purification step will be low in productivity, leading to industrial disadvantages.

The mixing of raw dimethyl sulfoxide and water can be carried out by a commonly used method. The entire amount of water may be added to raw dimethyl sulfoxide at a time or divided into several parts. Alternatively, the entire amount of raw dimethyl sulfoxide may be added to water at a time or divided into several parts.

For the purification method according to the present invention, the distillation of a mixed solution of raw dimethyl sulfoxide and water may be carried out in one stage or in two stages in order to remove the water to obtain dimethyl sulfoxide. When the distillation is carried out in two stages, the first stage is designed for evaporating water from a mixed solution of raw dimethyl sulfoxide and water to provide a roughly purified dimethyl sulfoxide and the second stage is designed for distilling the roughly purified dimethyl sulfoxide resulting from the first stage. Specifically, water is removed to provide roughly purified dimethyl sulfoxide in the first distillation stage and further purified dimethyl sulfoxide is obtained in the second distillation stage.

The distillation in the first stage may be performed by a commonly used distillation method such as, for example, high pressure distillation, low pressure distillation, reduced pressure distillation, molecular distillation, simple distillation, rectification, continuous distillation, and batch distillation, of which rectification under reduced pressure and simple distillation are preferable. The distillation in the second stage may be performed by high pressure distillation, low pressure distillation, reduced pressure distillation, molecular distillation, simple distillation, rectification, continuous distillation, batch distillation, or thin film distillation, of which rectification under reduced pressure, simple distillation, and thin film distillation are preferable.

In the purification method according to the present invention, the distillation of a mixed solution can be carried out in a single stage so that water is removed by evaporation to provide dimethyl sulfoxide. When the distillation is carried out in a single stage, the interior temperature is preferably 130°C or less, more preferably 100°C or less, and still more preferably 90°C or less.

In a purification process in which the distillation is carried out in two stages, the first distillation stage is designed for removing water from a mixed solution by evaporation to provide roughly purified dimethyl sulfoxide. The subsequent second distillation stage is designed for distilling the roughly purified dimethyl sulfoxide to provide purified dimethyl sulfoxide. For the first stage of this process, the interior temperature is preferably 130°C or less, more preferably 100°C or less, and still more preferably 90°C or less. For the second stage, the interior temperature is preferably 130°C or less, more preferably 110°C or less.

Reduced pressure distillation is performed preferably at a pressure 14.2 kPa or less, more preferably 6.7 kPa or less. If the distillation temperature or pressure is high, the decomposition of dimethyl sulfoxide will be accelerated, possibly making it impossible to depress the aggravation of odors.

The purification method described above can provide high-quality dimethyl sulfoxide that is conformable to the Pharmacopoeia and free of significant odors and has a very high chemical purity. It is preferable for this high purity dimethyl sulfoxide to have a purity of preferably 99.996% or more as determined by gas chromatograph (GC) analysis.

### Examples

The present invention will now be illustrated in more detail with reference to examples, but it should be understood that the invention is not construed as being limited to these examples.

For these examples, the chemical purity of raw dimethyl sulfoxide and the absorbance and odor intensity of dimethyl sulfoxide resulting from purification treatment (hereinafter referred to as purified dimethyl sulfoxide) and raw dimethyl sulfoxide were measured as described above.

### (Method for measuring the chemical purity of raw dimethyl sulfoxide)

The chemical purity of raw dimethyl sulfoxide was measured using a gas chromatograph (GC-2010, manufactured by Shimadzu) under the following conditions.
Analysis equipment: GC (GC-2010, manufactured by Shimadzu)
Carrier gas: helium (column flow rate 1.7 mL/min)
Analysis column: Rxt-1 (15 m × 0.32 mm × 3 µm) (manufactured by RESTEK)
Column temperature: 100°C (15 min) --> (10°C/min) --> 170°C (20 min)
Inlet temperature: 210°C
Injection volume: 1 µL
Detector: FID
Detector temperature: 220°C

### (Method for measuring the impurities in purified dimethyl sulfoxide)

Mass analysis was performed using a gas chromatograph (7890A, manufactured by Agilent) under the following conditions and unidentifiable impurities detected at a retention time of 4.3 minutes were examined.
Analysis equipment: GC (7890A, manufactured by Agilent)
Carrier gas: helium (column flow rate 2.0 mL/min)
Analysis column: Stabilwax (30 m × 0.32 mm × 0.5 µm) (manufactured by RESTEK)
Column temperature: 35°C (3 min) --> (7°C/min) --> 130°C (10 min) --> (7°C/min) --> 160°C --> (15°C/min) --> 250°C (8 min)
Inlet temperature: 200°C
Injection volume: 1 µL
Detector: FID
Detector temperature: 270°C

### (Method for measuring the absorbance of purified dimethyl sulfoxide and raw dimethyl sulfoxide)

Dimethyl sulfoxide was first subjected to nitrogen bubbling for 30 minutes and within 10 minutes, the absorbance at 275 nm was measured using an ultraviolet absorptiometer (UV-1800, manufactured by Shimadzu) under the following conditions.
Analysis equipment: ultraviolet absorptiometer
Cell: quartz, width 1 cm
Blank: water

### (Method for measuring the odor intensity of purified dimethyl sulfoxide and raw dimethyl sulfoxide)

Sensory evaluation of the odor of dimethyl sulfoxide was performed by three testers according to the following six-stage odor intensity criterion. In a 30 mL sample bottle, 15 mL of dimethyl sulfoxide was taken to provide a specimen. Each of the three testers brought the nose close to the sample bottle at room temperature, sniffed the odor from the same specimen, and ranked the odor intensity according to the following six-stage (rank 0 to 5) criterion. The rank agreed on by the largest number of testers was taken as the odor intensity of the specimen.
Odor intensity 0: odorless
Odor intensity 1: barely perceivable odor
Odor intensity 2: identifiable but weak order
Odor intensity 3: easily perceivable odor
Odor intensity 4: strong odor
Odor intensity 5: intense odor

### (Example 1)

In a 200 mL round bottom flask, 50 g of dimethyl sulfoxide of general industry grade with a purity of 99.9 wt% (absorbance 0.22 at 275 nm and odor intensity 3) and 50 g (100 parts by weight relative to 100 parts by weight of dimethyl sulfoxide) of ion-exchanged water conformable to A4 of JIS-K0557 were fed and mixed. A rectifying tube with a diameter of 3 cm and a height of 15 cm containing Raschig rings (each having a length of 5 mm, outside diameter of 5 mm, and inside diameter of 3 mm) installed over a total length of 10 cm was fitted on the mouth of the round bottom flask and distillation was performed at a reduced pressure of 6.7 kPa. Heating of the round bottom flask was started at an internal temperature of 45°C and the receiver was replaced when a temperature arrived to 107°C, thus providing 40 g of purified dimethyl sulfoxide. At this point, the distillation yield was 80%. The resulting purified dimethyl sulfoxide showed an absorbance at 275 nm of 0.12 and an odor intensity of 1. These results are summarized in Table 1. In the table, the quantity of water added is shown in terms of parts by weight relative to 100 parts by weight of dimethyl sulfoxide.

### (Examples 2 to 5)

Except for adding water in the amount shown in Table 1, the same purification procedure as in Example 1 was carried out. The distillation yield was 81%, 80%, 70%, and 79% in Examples 2, 3, 4, and 5, respectively. Qualities of the resulting purified dimethyl sulfoxide samples are described in Table 1.

### (Examples 6 and 7)

Except for using distilled water or ultrapure water conformable to A4 of JIS-K0557 instead of the water added in Example 1, the same purification procedure as in Example 1 was carried out. The distillation yield was 79% and 81% in Examples 6 and 7, respectively. Qualities of the resulting purified dimethyl sulfoxide samples are described in Table 1.

[Table 1]

**Table 1**

| | | | Example 1 | Example 2 | Example 3 | Example 4 | Example 5 | Example 6 | Example 7 |
|---|---|---|---|---|---|---|---|---|---|
| Purity of raw dimethyl sulfoxide | | wt% | 99.9 | 99.9 | 99.9 | 99.9 | 99.9 | 99.9 | 99.9 |
| Type of water | | (-) | ion-exchanged water | ion-exchanged water | ion-exchanged water | ion-exchanged water | ion-exchanged water | distilled water | ultrapure water |
| Amount of water added | | parts by weight | 100 | 50 | 200 | 40 | 35 | 100 | 100 |
| Internal temperature during distillation | | °C | 45-107 | 45-107 | 45-107 | 45-107 | 45-107 | 45-107 | 45-107 |
| Quality | Absorbance | (-) | 0.12 | 0.13 | 0.08 | 0.14 | 0.14 | 0.12 | 0.12 |
| | Odor intensity | (-) | 1 | 1 | 1 | 2 | 2 | 1 | 1 |

| | | | | | | | | | |
|---|---|---|---|---|---|---|---|---|---|
| Note) The ion-exchanged water, distilled water, and ultrapure water included here are all conformable to A4 of JIS K0557. | | | | | | | | | |

### (Comparative examples 1 to 4)

In Comparative examples 1, 2, and 4, the same procedure as in Example 1 was carried out except for adding water in the amount shown in Table 2. In Comparative example 3, the same procedure as in Example 1 was carried out except for adding methanol (guaranteed reagent, manufactured by Nacalai Tesque, Inc.) instead of water. The distillation yield was 85%, 95%, 95%, and 79% in Comparative examples 1, 2, 3, and 4, respectively. Qualities of the resulting dimethyl sulfoxide samples are described in Table 2.

[Table 2]

**Table 2**

| | | | Comparative example 1 | Comparative example 2 | Comparative example 3 | Comparative example 4 |
|---|---|---|---|---|---|---|
| Purity of raw dimethyl sulfoxide | | wt% | 99.9 | 99.9 | 99.9 | 99.9 |
| Type of liquid added | | (-) | ion-exchanged water | none | methanol | ion-exchanged water |
| Amount of liquid added | | parts by weight | 10 | 0 | 100 | 33 |
| Internal temperature during distillation | | °C | 45-107 | 45-107 | 45-107 | 45-107 |
| Quality | Absorbance | (-) | 0.16 | 0.22 | 0.21 | 0.14 |
| | Odor intensity | (-) | 3 | 3 | 3 | 3 |

| | | | | | | |
|---|---|---|---|---|---|---|
| Note) The ion-exchanged water included here is conformable to A4 of JIS K0557. | | | | | | |

### (Example 8)

In a 200 mL round bottom flask, 50 g of dimethyl sulfoxide of general industry grade with a purity of 99.9 wt% (absorbance 0.22 at 275 nm and odor intensity 3) and 50 g (100 parts by weight relative to 100 parts by weight of dimethyl sulfoxide) of tap water were fed and mixed. The tap water used had an electrical conductivity of 13 mS/m (25°C). A rectifying tube with a diameter of 3 cm and a height of 15 cm containing Raschig rings (each having a length of 5 mm, outside diameter of 5 mm, and inside diameter of 3 mm) installed over a total length of 10 cm was fitted on the mouth of the round bottom flask and distillation was performed at a reduced pressure of 6.7 kPa. The round bottom flask was heated from an internal temperature of 45°C to 80°C and the distillation was stopped at the time when the water content in the distillate came to 0.1% or below. The dimethyl sulfoxide left after removing water showed an absorbance at 275 nm of 0.11 and an odor intensity of 1. These results are summarized in Table 3. In the table, the quantity of water added is shown in terms of parts by weight relative to 100 parts by weight of dimethyl sulfoxide.

### (Examples 9 and 10)

Except for adding water in the amount shown in Table 3, the same distillation procedure as in Example 8 was carried out. Qualities of the resulting dimethyl sulfoxide samples are described in Table 3.

### (Examples 11 and 12)

After heating the round bottom flask from an internal temperature of 45°C to 70°C, simple distillation was performed instead of using a rectifying tube as in Example 8 and water was added in the amount described in Table 3. Except for these changes, the same distillation procedure as in Example 8 was carried out. Qualities of the resulting dimethyl sulfoxide samples are described in Table 3.

[Table 3]

**Table 3**

| | | | Example 8 | Example 9 | Example 10 | Example 11 | Example 12 |
|---|---|---|---|---|---|---|---|
| Purity of raw dimethyl sulfoxide | | wt% | 99.9 | 99.9 | 99.9 | 99.9 | 99.9 |
| Type of water | | (-) | tap water | tap water | tap water | tap water | tap water |
| Amount of water added | | parts by weight | 100 | 150 | 200 | 150 | 200 |
| Distillation conditions | | (-) | rectification | rectification | rectification | simple distillation | simple distillation |
| Internal temperature during distillation | | °C | 45-80 | 45-80 | 45-80 | 45-70 | 45-70 |
| Quality | Absorbance | (-) | 0.11 | 0.10 | 0.12 | 0.10 | 0.12 |
| | Odor intensity | (-) | 1 | 1 | 1 | 1 | 1 |

### (Example 13)

In a 200 mL round bottom flask, 50 g of dimethyl sulfoxide of general industry grade with a purity of 99.9 wt% (absorbance 0.22 at 275 nm and odor intensity 3) and 50 g (100 parts by weight relative to 100 parts by weight of dimethyl sulfoxide) of tap water were fed to prepare a mixed solution. A rectifying tube with a diameter of 3 cm and a height of 15 cm containing Raschig rings (each having a length of 5 mm, outside diameter of 5 mm, and inside diameter of 3 mm) installed over a total length of 10 cm was fitted on the mouth of the round bottom flask and distillation was performed at a reduced pressure of 6.7 kPa as the first-stage distillation for the mixed solution. The round bottom flask was heated from an internal temperature of 45°C to 80°C and the distillation was stopped at the time when the water content in the distillate came to 0.1% or below. The roughly purified dimethyl sulfoxide left after removing water showed an absorbance at 275 nm of 0.11 and an odor intensity of 1.

For the second-stage distillation, the roughly purified dimethyl sulfoxide obtained from the first-stage distillation was fed in a 200 mL round bottom flask and a rectifying tube with a diameter of 3 cm and a height of 15 cm containing Raschig rings (each having a length of 5 mm, outside diameter of 5 mm, and inside diameter of 3 mm) installed over a total length of 10 cm was fitted, followed by heating of the round bottom flask from an internal temperature of 45°C to 107°C and distillation at a reduced pressure of 6.7 kPa. The purified dimethyl sulfoxide obtained from the second-stage distillation showed an absorbance at 275 nm of 0.09 and an odor intensity of 1. These results are summarized in Table 4.

### (Example 14)

Except for adding water in the amount shown in Table 4 instead of the amount adopted in Example 13, the same first- and second-stage distillation procedures as in Example 13 were carried out. The roughly purified dimethyl sulfoxide obtained from the first-stage distillation showed an absorbance at 275 nm of 0.10, an odor intensity of 1, and a chemical purity of 99.9957% as determined by GC analysis. The purified dimethyl sulfoxide obtained from the second-stage distillation showed an absorbance at 275 nm of 0.08, an odor intensity of 1, and a chemical purity of 99.9989% as determined by GC analysis. These results are summarized in Table 4.

### (Example 15)

Except for adding water in the amount shown in Table 4 instead of the amount adopted in Example 13, the same first- and second-stage distillation procedures as in Example 13 were carried out. The roughly purified dimethyl sulfoxide obtained from the first-stage distillation showed an absorbance at 275 nm of 0.12, an odor intensity of 1, and a chemical purity of 99.9957% as determined by GC analysis. The purified dimethyl sulfoxide obtained from the second-stage distillation showed an absorbance at 275 nm of 0.08, an odor intensity of 1, and a chemical purity of 99.9980% as determined by GC analysis. These results are summarized in Table 4.

### (Example 16)

Water was added in the amount shown in Table 4 instead of the amount adopted in Example 13; no rectifying tube was used in the first-stage distillation procedure, in which simple distillation was performed after heating the round bottom flask from an internal temperature of 45°C to 70°C; and no rectifying tube was used in the second-stage distillation procedure, in which simple distillation was performed after heating the round bottom flask from an internal temperature of 45°C to 80°C. Except for these changes, the same distillation procedures as in Example 13 were carried out. Qualities of the resulting purified dimethyl sulfoxide sample are described in Table 4.

### (Example 17)

Except for adding water in the amount shown in Table 4 instead of the amount adopted in Example 16, the same first- and second-stage distillation procedures as in Example 16 were carried out. Qualities of the resulting purified dimethyl sulfoxide sample are described in Table 4.

### (Example 18)

Water was added in the amount shown in Table 4 instead of the amount adopted in Example 13, and no rectifying tube was used in the second-stage distillation procedure in which thin film distillation was performed after heating the round bottom flask from an internal temperature of 80°C to 110°C. Except for these changes, the same distillation procedures as in Example 13 were carried out. Qualities of the resulting purified dimethyl sulfoxide sample are described in Table 4.

[Table 4]

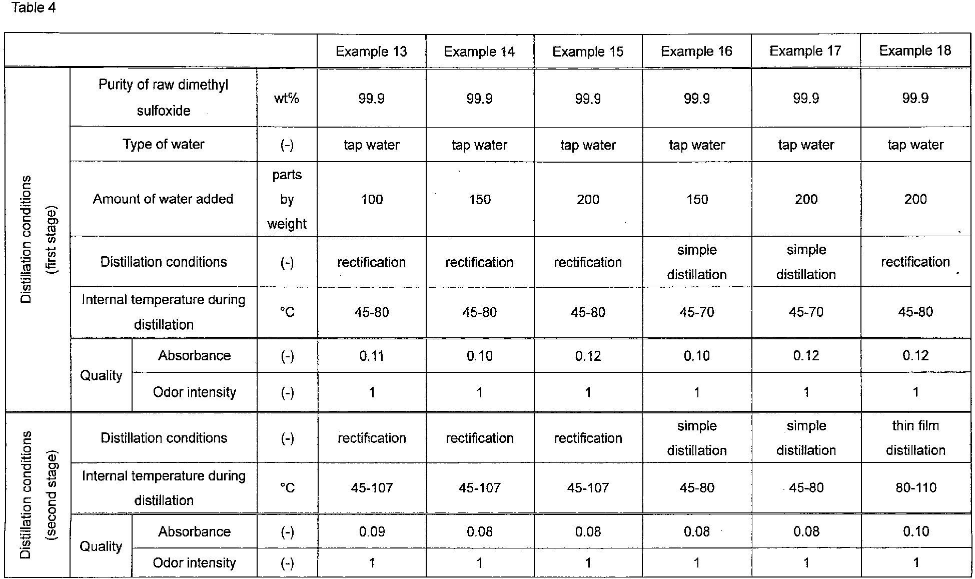

## Claims

1. A method for purifying dimethyl sulfoxide including a step for distilling a mixed solution prepared by adding 35 parts by weight or more of water to 100 parts by weight of raw dimethyl sulfoxide.

2. A method for purifying dimethyl sulfoxide as described in claim 1 wherein the purified dimethyl sulfoxide has an absorbance of 0.01 to 0.20 at 275 nm.

3. A method for purifying dimethyl sulfoxide as described in either claim 1 or 2 wherein the water is ion-exchanged water, distilled water, ultrapure water, or tap water.

4. A method for purifying dimethyl sulfoxide as described in any one of claims 1 to 3 wherein the water has an electrical conductivity of 50 mS/m or less at 25°C.

5. A method for purifying dimethyl sulfoxide as described in any one of claims 1 to 4 wherein the distillation of a mixed solution is carried out in a single step for evaporating water to obtain dimethyl sulfoxide.

6. A method for purifying dimethyl sulfoxide as described in any one of claims 1 to 4 wherein the distillation of a mixed solution is carried out in a first step for evaporating water to obtain roughly purified dimethyl sulfoxide, followed by a second step for distilling the roughly purified dimethyl sulfoxide to obtain purified dimethyl sulfoxide.

7. High-purity dimethyl sulfoxide obtained by a purification method as described in any one of claims 1 to 6 and having a purity of 99.996% or more as determined by gas chromatograph (GC) analysis.
